# EUROPEAN PATENT APPLICATION

(11) **EP 2 497 490 A1**
(43) Date of publication of application: **12.09.2012**
(21) Application number: 11305236.9
(22) Date of filing: 07.03.2011
(51) Int. Cl.: A61K 38/55, A61P 15/08, G01N 33/50

(54) **Methods for diagnosing and/or treating sterility**

(71) Applicant: Bioquanta, 75005 Paris (FR); Bioquanta Corp., Aurora, CO 80014 (US); ASSISTANCE PUBLIQUE - HOPITAUX DE PARIS, 75001 Paris (FR)
(72) Inventor: Conti, Marc, 91120, Palaiseau (FR); Loric, Sylvain, 91800, Brunoy (FR); Manivet, Philippe, 75012, Paris (FR); Caradec, Josselin, 75015, Paris (FR); Keumeugni Kwemo Carlosse, 92390, Villeneuve la Garénne (FR); Matar, Corine, 75008, Paris (FR); Revaud, Déborah, 91590, Mondeville (FR)
(74) Representative: Blot, Philippe Robert Emile

(57) **Abstract**

The present invention concerns a polypeptide comprising the N-terminal fragment of a cystatin, and/or a cystatin expression inducer for use for the treatment of sterility and/or infertility, and a method of diagnosing and/or predicting sterility and/or infertility of a subject and/or of a couple of subjects, which method comprises measuring the level of at least one cystatin in a biological sample of a male subject and/or measuring the level of at least one cystatin in a biological sample of a female subject.

SEQUENCE LISTING

<110>  BIOQUANTA
       BIOQUANTA CORP.
       ASSISTANCE PUBLIQUE - HOPITAUX DE PARIS

<120>  Methods for diagnosing and/or treating sterility

<130>  BET10P1431

<160>  2

<170>  PatentIn version 3.4

<210>  1
<211>  146
<212>  PRT
<213>  Homo sapiens

<400>  1

Met Ala Gly Pro Leu Arg Ala Pro Leu Leu Leu Leu Ala Ile Leu Ala
1               5                   10                  15

Val Ala Leu Ala Val Ser Pro Ala Ala Gly Ser Ser Pro Gly Lys Pro
            20                  25                  30

Pro Arg Leu Val Gly Gly Pro Met Asp Ala Ser Val Glu Glu Glu Gly
        35                  40                  45

Val Arg Arg Ala Leu Asp Phe Ala Val Gly Glu Tyr Asn Lys Ala Ser
    50                  55                  60

Asn Asp Met Tyr His Ser Arg Ala Leu Gln Val Val Arg Ala Arg Lys
65                  70                  75                  80

Gln Ile Val Ala Gly Val Asn Tyr Phe Leu Asp Val Glu Leu Gly Arg
                85                  90                  95

Thr Thr Cys Thr Lys Thr Gln Pro Asn Leu Asp Asn Cys Pro Phe His
            100                 105                 110

Asp Gln Pro His Leu Lys Arg Lys Ala Phe Cys Ser Phe Gln Ile Tyr
        115                 120                 125

Ala Val Pro Trp Gln Gly Thr Met Thr Leu Ser Lys Ser Thr Cys Gln
    130                 135                 140

Asp Ala
145

<210>  2
<211>  9
<212>  PRT
<213>  Artificial

<220>
<223>  N-terminal fragment of cystatin 3

<400>  2

Ser Ser Pro Gly Lys Pro Pro Arg Leu
1               5

## Description

The present invention concerns methods for diagnosing and/or treating sterility and/or infertility in mammals. In particular, the present invention concerns a polypeptide comprising the N-terminal fragment of a cystatin, and/or a cystatin expression inducer for use for the treatment of sterility and/or infertility, and a method of diagnosing and/or predicting sterility and/or infertility of a subject and/or of a couple of subjects, which method comprises measuring the level of at least one cystatin in a biological sample of a male subject and/or measuring the level of at least one cystatin in a biological sample of a female subject.

Human infertility is defined by the Word Health Organization as the inability of a couple to conceive within two years of exposure to pregnancy, the expression "exposure to pregnancy" referring to women of reproductive age (15 to 49) at risk of pregnancy (not pregnant, sexually active, non-contracepting and nonlactating).

By this definition, more than 70 million couples worldwide are infertile, which represents 13% to 15% of couples over the world. The prevalence varies widely, being less in developed countries and more in developing countries where limited resources for investigation and treatment are available. In France, about 15% of couples have fertility problems.

In addition, infertility is considered as a public problem because it not only affects the couples' life, but also the healthcare services and social environment. Indeed, the feelings experienced by the infertile couples include depression, grief, guilt, shame and inadequacy with social isolation (Kamel (2010) Reprod. Biol. Endocrin. 8:21).

Among the couples who have fertility problems, about 30% of the infertilities have a female origin and are linked to ovulation disorders or tubal infertility; 20% of the infertilities have a male origin; 40% are of mixed origin and 10% are unexplained. In the cases of unexplained infertilities, no abnormality of the reproductive function can be established in either the male or female partner.

Accordingly, there is still a need for methods of diagnosis and treatment of infertility, in particular of unexplained infertility.

The present invention arises from the unexpected finding by the inventors that cystatin 3 plays a key role in the protection of spermatozoa in the vagina after the intercourse.

Cystatins (CST) form a superfamily of structurally related proteins with different molecular properties and biological distribution. They are all potent, reversible, competitive inhibitors of cysteine proteases such as papain and many human cathepsins (Abrahamson et al. (2003) Biochem. Soc. Symp. 70:179-199). Cathepsins (CTS) are a family of lysosomal proteinases active in an acidic environment. They have the ability to degrade extracellular matrix molecules, including collagens, laminin, fibronectin and proteoglycans and are also involved in the catabolism of intracellular proteins and prohormone processing. The cathepsins are mainly lysosomal proteases that can degrade extracellular matrix and basement membrane proteins (Buck et al. (1992) Biochem. J. 282:273-278) and some of these enzymes are also thought to be involved in the pathogenesis of inflammatory and malignant diseases. The balance between these enzymes and their inhibitors (e.g. cystatins) is thus most important for protection against endogenous proteolysis.

The cystatin protein superfamily contains three major types of inhibitors:
- type 1 cystatins represent intracellular protease inhibitors, but low levels of these are also found in some body fluids (Abrahamson et al. (1986) J. Biol. Chem. 261:11282-11289);
- type 2 cystatins probably have predominantly extracellular functions because they are synthesized with signal peptides for secretion (Abrahamson et al. (2003) Biochem. Soc. Symp. 70:179-199). Among the members of the type 2 cystatins, cystatin C displays the strongest inhibitory activity of all cystatins towards lysosomal cysteine proteases in general and has a widespread distribution in human tissues and body fluids (Abrahamson et al. (1986) J. Biol. Chem. 261:11282-11289); and
- type 3 cystatins, together with cystatin C and α₂-macroglobulin, are the most important plasma inhibitors of cysteine proteases (Abrahamson et al. (1986) J. Biol. Chem. 261:11282-11289).

Among cystatins, cystatin C, also called cystatin 3 (CST3) is one of the main extracellular inhibitors of the papain-like cathepsins. CST3 inhibits family C1 cysteine proteases through tight and reversible binding in a substrate-competing mechanism resulting in an equimolar complex with the protease (Abrahamson et al. (1987) J. Biol. Chem. 262:9688-9694).

Expression of CST3 is reported in all human organs, and CST3 is detected in many biological fluids, the highest concentrations being found in seminal plasma, cerebrospinal fluid, and synovial fluid (Abrahamson et al. (1986) J. Biol. Chem. 261:11282-11289). CST3 was also reported to be highly expressed in the human male reproductive tract (Jiborn et al. (2004) J. Androl. 25:564-572). However, mRNA levels vary greatly between different tissue types (Abrahamson et al. (1990) Biochem. J. 268:287-294). CST3 is in particular widely distributed in normal tissues from the testis, epididymis, vas deferens, seminal vesicle, and prostate gland. Immunoreactive CST3 was localized in basal and secretory epithelial cells, but also in neuroendocrine cells in the prostate.

Owing to its small size (13.3 kDa) and steady production, the serum level of cystatin C is a reliable marker for the glomerular filtration rate (GFR) in the non-pregnant setting (Grubb (2000) Adv. Clin. Chem. 35:63-99). The activity of CST3 can be downregulated in inflammatory conditions by the serine protease, neutrophil elastase (Abrahamson et al. (1991) Biochem. J. 273:621-626). Like other members of the family 2 cystatins (e.g. cystatin D, S, SA, and SN), CST3 is reported to regulate cysteine proteinase action during normal body processes including bone resorption (Lerner and Grubb (1992) J. Bone Mineral Res. 7:433-440) and tissue remodeling. CST3 deficiency and increased cysteine proteinase activity is associated with numerous pathological conditions associated with extracellular matrix degradation including cancer progression and atherosclerosis (Shi et al. (1999) J. Clin. Invest. 104:1191-1197).

In female humans, CTSB, CTSH, CTSK, CTSL, and CTSS are expressed in the proliferative and secretory phase endometria and appear to be required for normal uterine development and function as well as menstruation (Jokimaa et al. (2001) Mol. Hum. Reproduction 7:73-78). CTSD (Camier et al. (1996) Hum. Reproduction 11:392-397), CTSH (Allan et al. (2003) Endocr. Res. 29:53-65), CTSB, CTSL and CTSS (Jokimaa et al. (2001) Mol. Hum. Reproduction 7:73-78) were identified as displaying a differential regulation in primate endometrium during the menstrual cycle. These findings have suggested that cathepsins are needed for normal development and function of human endometrium during both the proliferative and secretory phases.

On the other hand, CSTs have been very poorly studied in vertebrate follicular or endometrium development. Only two articles so far have reported CST3 expression in the macaque vagina : Slayden et al. (2004 J. Clin. Endocrinol. Metab. 89:883-891) revealed by *in situ* hybridization and immunocytochemistry that CST3 was localized in fibroblasts and smooth muscle cells of the vagina. Estradiol increased vaginal CST3 expression in the fibroblasts and smooth muscle bundles, and progesterone suppressed this effect. More recently, CST3 expression has been studied during the cycle of non pregnant ewes, and compared to pregnant ones (Song et al. (2006) Endocrinology 147:3478-3483). *In situ* hybridization and immunocytochemistry analyses to determine the location of both *CST3* mRNA and protein in the uterus have revealed that CST3 is present only in both luminal (LE) and glandular epithelium (GE) of the endometrium. Consistent with immunohistochemical studies, CST3 protein was detected predominantly in uterine flushes from day 12 of cyclic ewes suggesting that CST3 is produced around the ovulatory peak.

Available evidences support the concept that a variety of proteases as well as their specific inhibitors regulate trophoblast invasion in many species (e.g. mouse, rat, cat, pig, and human) during conceptus implantation (Jokimaa et al. (2001) Mol. Hum. Reproduction 7:73-78; Afonso et al. (1997) Development 124:3415-3425; Elangovan and Moulson (1980) Biol. Reprod. 23:663-668; Li et al. (1991) Biol. Reprod. 44:625-631; Li et al. (1992) Biol. Reprod. 47:21-28). Specifically, these studies implicate CTS in regulation of uterine receptivity for implantation and trophoblast invasion in a number of mammals (Salamonsen and Wooley (1996) Human Reproduction 11:124-133; Curry and Osteen (2003) Endocr. Rev. 24:428-465).

CST3 expression in the ovine uterus is similar to that reported for mice (Afonso et al. (1997) Development 124:3415-3425), in which expression of CTSL and CTSB by invasive trophoblast giant cells was balanced by coordinated expression of CST3 in the decidualizing stroma at the implantation site (Song et al. (2006) Endocrinology 147:3478-3483).

Accordingly, the above previous studies demonstrated a potential role of CST3 in maturation of sperm and in implantation and nidation, but they do not suggest that CST3 could affect the fecundation step, in particular both the step of movement of the spermatozoa from the vagina to the fallopian tubes and the step of fusion of the ovum with a spermazoid.

The inventors demonstrated that the presence of CST3 in very high concentration in semen of non-sterile males protects sperm against cathepsins when seminal plasma is released within the vagina. The higher the amount of CST3 in seminal plasma, the better the protection conferred against vaginal secreted cathepsins. Without being bound by this theory, it is thought that patients with low CST3 level would not be able to reduce efficiently female host response after intercourse and are likely to be sterile by excessive germ cell destruction. The inventors indeed demonstrated that 30% of sterile men displayed a concentration of CST3 in semen which was low to very low, that is to say less than 30 ng/ml, and which induced a disruption of the proteinase/antiproteinase balance. The same holds true if CST3 secreted by vagina and endometrium is too low to limit cathepsins activity against male germ cells.

The invention thus relates to a method for treating sterility and/or infertility by increasing at least one CST activity in the vagina. In particular, a polypeptide comprising the N-terminal fragment of a cystatin, and/or a cystatin expression inducer may be used for the treatment of sterility and/or infertility.

The present invention also concerns an *in vitro* method of diagnosing and/or predicting sterility and/or infertility of a subject and/or of a couple of subjects comprising the steps of:
(i) measuring the level of at least one cystatin in a biological sample of a male subject, wherein said sample is selected from the group consisting of blood, plasma, serum, semen, sperm and seminal plasma, and/or
(ii) measuring the level of at least one cystatin in a biological sample of a female subject, wherein said sample is selected from the group consisting of blood, plasma, serum, cervical mucus and vaginal mucus, and
(iii) comparing the level of at least one cystatin measured in step (i) and/or (ii) with a male control level of said at least one cystatin or female control level of said at least one cystatin, as appropriate;
wherein a decreased level of the at least one cystatin measured in step (i) and/or (ii) compared to said male control level of said at least one cystatin or female control level of said at least one cystatin is indicative of sterility and/or infertility of said subject and/or couple of subjects, or is indicative of a higher risk of sterility and/or infertility of said subject and/or couple of subjects.

### Detailed description of the invention

### Cystatins and cystatins expression inducer

In the context of the invention, the Swiss Prot and Genbank references cited hereinafter are those that were available on December 1 st, 2010.

As known from the skilled person, cystatins are a family of cysteine protease inhibitors with homology to chicken cystatin. Cystatins typically comprise about 145 amino acids, are largely acidic, contain four conserved cysteine residues known to form two disulfide bonds, may be glycosylated and/or phosphorylated, with similarity to fetuins, kininogens, stefins, histidine-rich glycoproteins and cystatin-related proteins. They mainly inhibit peptidases belonging to peptidase families C1 (papain family) and C13 (legumain family) (Turk and Bode (1991) FEBS Letters 285:213-219).

The cystatin family includes:
- type 1 cystatins, which are intracellular cystatins that are present in the cytosol of many cell types, but can also appear in body fluids at significant concentrations. They are single-chain polypeptides of about 100 residues, which have neither disulphide bonds nor carbohydrate side chains.
- type 2 cystatins, which are mainly extracellular secreted polypeptides synthesised with a 19-28 residue signal peptide. They are broadly distributed and found in most body fluids. Type 2 cystatins include in particular cystatin C.
- type 3 cystatins, which are multidomain proteins.
- unclassified cystatins, which are cystatin-like proteins found in a range of organisms, and include plant phytocystatins, fetuin in mammals, insect cystatins and a puff adder venom cystatin which inhibits metalloproteases of the MEROPS peptidase family M12 (astacin/adamalysin).

In the context of the invention, the cystatin is preferably a type 2 cystatin, as defined above. More preferably, the cystatin is cystatin C.

Cystatin C, also called cystatin 3, gamma trace, post-gamma-globulin, neuroendocrine basic polypeptide or CST3, is a protein encoded by the *CST3* gene. The sequence of the human *CST3* gene is referenced under Genbank accession number n° X52255. Cystatin 3 protein is typically constituted of 146 amino acids. CST3 is synthesized in a precursor form including a signal peptide corresponding to amino acids 1-26 and a mature chain corresponding to amino acids 27-146. The amino acid sequence of the precursor form of human cystatin 3 is for example referenced under Swiss Prot Number P01 034 and is typically as shown in SEQ ID NO: 1. As used hereinafter, the term "cystatin 3 protein" or "CST3" corresponds to the mature chain of cystatin 3, preferably to the mature chain of human cystatin 3.

Several studies demonstrated that the N-terminal domain of CST3 is necessary to observe the cystein protease inhibitory activity of CST3 (Abrahamson *et al.* (1987) *J. Biol. Chem.* **262**:9688-9694; Abrahamson *et al.* (1991) *Biochem. J*. **273**:621-626). This N-terminal domain (or N-terminal fragment) of CST3 consists of the sequence SSPGKPPRL (SEQ ID NO: 2) and corresponds to amino acids 27-35 of the precursor form of CST3 or to amino acids 1-9 of mature CST3.

In a preferred embodiment, the polypeptide comprising the N-terminal fragment of a cystatin is a fragment of a mature cystatin protein, in particular a fragment of a mature type 2 cystatin, more particularly a fragment of the mature CST3 protein, or a fragment of the precursor form of a cystatin, in particular a fragment of the precursor form of a type 2 cystatin, more particularly a fragment of the precursor form of CST3.

In another preferred embodiment, the polypeptide comprising the N-terminal fragment of a cystatin is a mature cystatin protein, in particular a mature type 2 cystatin, more particularly the mature CST3 protein, or the presursor form of a cystatin, in particular the precursor form of a type 2 cystatin, more particularly the precursor form of CST3. Preferably, the polypeptide comprising the N-terminal fragment of a cystatin is a mature cystatin protein, in particular a mature type 2 cystatin, more particularly the mature CST3 protein.

In the context of the invention, the terms "polypeptide" and "peptide" are used indifferently and refer to native peptides (either purified or recombinant products, or synthetically synthesized peptides) and further to modified peptides, or peptidomimetics, such as peptoids and semipeptoids. Polypeptides include amino acid sequences modified either by natural processes, such as post-translational processing, or by chemical modification techniques that are well known in the art. Such modifications are well described in the literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications.

Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, γ-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, sumoylation and ubiquitination. These modifications are for example described in "Proteins - Structure and molecular properties", 2nd Ed., Creighton, T.E., Freeman, W.H. et al., New York, 1993; Wold, F., "Post-translational protein modifications: perspectives and prospects", p1-12 in Post-translational covalent modification of proteins Johnson, B.C., Academic Press, New York, 1983; Seifter et al. (1990) Meth. Enzymol. 182:626-646; and Rattan et al. (1992) Ann NY Acad Sci 663:48-62.

Peptoids and semipeptoids are peptide analogs, which may have, for example, modifications rendering the peptides more stable while in a body, or more immunogenic. Such modifications include, but are not limited to, cyclization, N-terminus modification, C-terminus modification, peptide bond modification, including, but not limited to, CH₂-NH, CH₂-S, CH₂-S=O, O=C-NH, CH₂-O, CH₂-CH₂, S=C-NH, CH=CH or CF=CH, backbone modification and residue modification. Methods for preparing peptidomimetic compounds are well known in the art and are specified in Quantitative Drug Design, CA. Ramsden Gd., Chapter 17.2, F. Choplin Pergamon Press (1992).

In particular, the polypeptide according to the invention may be a polypeptide modified as described above, in such a way that it may be differentiated from the corresponding endogenous polypeptide. Such modified polypeptides include polypeptides comprising N¹⁵ or C¹³ atoms, which are distinguishable by mass spectrometry from natural polypeptides who comprise N¹⁴ and C¹² atoms.

According to the invention, a polypeptide consists of at least 9 amino acids, preferably at least 10 amino acids, more preferably at least 20 amino acids, still preferably at least 30 amino acids, still preferably at least 40 amino acids, still preferably at least 50 amino acids, still preferably at least 60 amino acids, still preferably at least 70 amino acids, still preferably at least 80 amino acids, still preferably at least 90 amino acids, still preferably at least 100 amino acids, still preferably at least 110 amino acids, still preferably at least 120 amino acids. More preferably, polypeptides according to the invention have a length of from about 9 to about 146 amino acids, from about 10 to about 140 amino acids, from about 20 to about 130 amino acids, from about 30 to about 120 amino acids, from about 40 to about 110 amino acids, from about 50 to about 100 amino acids. Most preferably, polypeptides according to the invention have a length of 120 amino acids.

As used herein, the term "amino acid" is understood to include: the 20 naturally occurring amino acids, i.e. alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine; amino acids harbouring the post-translational modifications which can be found *in vivo* such as hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids. The term "amino acid" also includes modified amino acids, in particular chemically modified amino acids, including but not limited to salts, amino acid derivatives (such as amides), substitutions, and/or those modified through chemical reactions and/or biosynthetically. Amino acids, including carboxy- and/or amino-terminal amino acids in peptides, can be modified by methylation, amidation, acetylation, protecting groups, and/or substitution with other chemical groups that can change the peptide's circulating half-life without adversely affecting their activity. Amino acids may comprise one or more posttranslational modifications, such as association with one or more chemical entities (e.g., methyl groups, acetate groups, acetyl groups, phosphate groups, formyl moieties, isoprenoid groups, sulfate groups, polyethylene glycol moieties, lipid moieties, carbohydrate moieties, biotin moieties, etc.).

In the context of the invention, the expression "cystatin expression inducer" refers to a compound that induces and/or increases the expression of a CST protein. In a preferred embodiment, the cystatin expression inducer according to the invention is a type 2 cystatin expression inducer. More preferably, said cystatin expression inducer is a cystatin 3 expression inducer.

Cystatin expression inducers, and in particular CST3 expression inducers are well-known from the skilled person and include for example oestradiol, progesterone (Song et al. (2006) Endocrinology 147:3478-3483), interferon-τ (Song et al. (2006) Endocrinology 147:3478-3483); interferon-β (Staun-Ram and Miller (2010) J. Neuroimmunol)*,* interferon-γ (Spencer et al. (2007) Reprod. Fertil. Dev. 19:65-78); TGF-β (Solem et al. (1990) Biochem. Biophys. Res. Commun. 172:945-951), glucocorticoids in particular dexamethasone (Bjarnadóttir et al. (1995) Scand. J. Clin. Lab. Invest. 55:617-623); cyclosporine A (Tsai et al. (2009) J. Periodontal. Res. 44:459-464) and 6-hydroxydopamine (Lee et al. (2006) NeuroToxicology 27:260-276). Accordingly, preferably, the cystatin expression inducer is selected from the group consisting of oestradiol, progesterone, interferon-τ, interferon-β, interferon-γ, TGF-β, glucocorticoids such as dexamethasone, cyclosporine A and 6-hydroxydopamine.

Assays for identifying cystatin expression inducers are well-known from the skilled person. Typically, such an assay includes contacting endometrial cells, in particular endometrial cancer cell lines such as Ishikawa cells or ECC-1 cell lines (Mo et al. (2006) Biol. Reprod. 75:387-394), with a candidate compound and detecting the release of cystatin by said endometrial cells

### Subject

In the context of the present invention, a "subject" denotes a human or non-human mammal, such as a rodent (rat, mouse, rabbit), a primate (chimpanzee), a feline (cat), a canine (dog), a bovine (cow), an ovine (ewe), a caprine (goat), an equid (horse). Preferably, the subject is human.

The subject according to the invention may be a male mammal or a female mammal.

The term "male" is defined herein as the sex of an organism, which produces spermatozoa. Each spermatozoon can fuse with an ovum, in the process of fertilization. A male cannot reproduce sexually without access to at least one ovum from a female.

The term "female" is defined herein as the sex of an organism, or a part of an organism, which produces ova. A female mammal cannot reproduce sexually without access to the gametes of a male. Preferably, the female mammal according to the invention is a female mammal of reproductive age at risk of pregnancy, i.e. not pregnant, sexually active, non-contracepting and non-lactating.

Preferably, the subject according to the invention is a male human, *i.e.* a man or a female human, *i.e.* a woman.

As used herein, the expression "couple of subjects" refers to a couple constituted by a male mammal and a female mammal who have intercourse. Preferably, the couple of subjects according to the invention is constituted by a man and a woman who have intercourse.

### Methods of treatment

The present invention relates to a polypeptide comprising N-terminal fragment of a cystatin, and/or a cystatin expression inducer, as defined above, for use for the treatment of sterility and/or infertility.

In a particular embodiment, the invention relates to a polypeptide comprising N-terminal fragment of a type 2 cystatin, in particular N-terminal fragment of cystatin 3, and/or a type 2 cystatin expression inducer, in particular a cystatin 3 expression inducer, as defined above, for use for the treatment of sterility and/or infertility.

As used herein, the term "infertility" corresponds to one year of unwanted non-conception with unprotected intercourse in the fertile phase of the menstrual cycles. Infertility encompasses primary infertility and secondary infertility.

As used herein, the term "primary infertility" refers to females who have never achieved pregnancy in the past.

As used herein, the term "secondary infertility" refers to females who have achieved pregnancy and given birth in the past, but are now having difficulty conceiving.

As used herein, the term "sterility" refers to a complete lack of fertility. According to the present invention, a person who is sterile has no potential to produce offspring ever, while a person who is infertile may be able to get pregnant over time. In the context of the invention, sterility may be male and/or female sterility.

As used herein, the term "male sterility" refers to the sterility as defined above of a male subject. Male sterility causes include:
- pre-testicular causes, *i.e.* conditions that impede adequate support of the testes and include situations of poor hormonal support and poor general health including hypogonadism; drugs, alcohol, smoking; strenuous riding; medications, including those that affect spermatogenesis such as chemotherapy, anabolic steroids, cimetidine, spironolactone, those that decrease FSH levels such as phenytoin, those that decrease sperm motility such as sulfasalazine and nitrofurantoin; genetic abnormalities such as a Robertsonian translocation;
- testicular factors, i.e. conditions where the testes produce semen of low quantity and/or poor quality despite adequate hormonal support and that include age; genetic defects on the Y chromosome; abnormal set of chromosomes such as Klinefelter syndrome; neoplasm such as seminoma; idiopathic failure; cryptorchidism; varicocele; trauma; hydrocele; mumps; malaria; testicular dysgenesis syndrome; acrosomal defects affecting egg penetration; idiopathic oligospermia;
- post-testicular causes, *i.e*. conditions that affect the male genital system after testicular sperm production and that include defects of the genital tract as well as problems in ejaculation such as vas deferens obstruction; lack of vas deferens; prostatitis; anti-sperm antibodies; retrograde ejaculation; ejaculatory duct obstruction; hypospadias; impotence.

As used herein, the term "female sterility" refers to the sterility as defined above of a female subject. Female sterility causes include:
- hypothalamic-pituitary factors, such as hypothalamic dysfunction and hyperprolactinemia;
- ovarian factors, such as polycystic ovarian syndrome; anovulation; diminished ovarian reserve; premature menopause; menopause; luteal dysfunction; gonadal dysgenesis (Turner syndrome); ovarian cancer;
- tubal or peritoneal factors such as endometriosis; pelvic adhesions; pelvic inflammatory disease; tubal occlusion; tubal dysfunction;
- uterine factors such as uterine malformations; uterine fibroids; Asherman's syndrome;
- cervical factors such as cervical stenosis; antisperm antibodies; non-receptive cervical mucus;
- vaginal factors such as vaginismus; vaginal obstruction;
- genetic factors such as androgen insensitivity syndrome.

The present inventors demonstrated that a given level of cystatin both from the male and the female was necessary, first to protect spermatozoa from female proteinases and then to ensure that egg fertilization will occur.

Therefore, the polypeptide comprising N-terminal fragment of a cystatin or the cystatin expression inducer according to the invention may also be for use for treating sterility and/or infertility of a couple of subjects.

In particular, the polypeptide comprising N-terminal fragment of a type 2 cystatin, more particularly of cystatin 3, or the type 2 cystatin expression inducer, more particularly the cystatin 3 expression inducer, according to the invention may be for use for treating sterility and/or infertility of a couple of subjects.

The present invention also relates to a method of treatment of sterility and/or infertility in a subject and/or a couple of subjects, comprising administering a therapeutically effective amount of (i) a polypeptide comprising N-terminal fragment of a cystatin, in particular of a type 2 cystatin, more particularly of cystatin 3 and/or of (ii) a cystatin expression inducer, in particular a type 2 cystatin expression inducer, more particularly a cystatin 3 expression inducer, as defined above to a subject and/or a couple of subjects in need thereof.

The present invention also relates to the use of (i) the polypeptide comprising N-terminal fragment of a cystatin, in particular of a type 2 cystatin, more particularly of cystatin 3, and/or of (ii) a cystatin expression inducer, in particular a type 2 cystatin expression inducer, more particularly a cystatin 3 expression inducer, according to the invention for the manufacture of a medicament intended for treating sterility and/or infertility in a subject and/or a couple of subjects.

In the context of the invention, the term "treating" or "treatment" means reversing, alleviating, inhibiting the progress of, or preventing the disorder or condition to which such term applies, or one or more symptoms of such disorder or condition.

In a particular embodiment, the polypeptide comprising N-terminal fragment of a cystatin is administered in combination, for simultaneous, separate or sequential administration, with at least one N-terminal fragment of another cystatin. In particular, the invention may relate to a polypeptide comprising N-terminal fragment of cystatin 3 or to the N-terminal fragment of cystatin 3 for use for the treatment of sterility and/or infertility wherein said polypeptide comprising N-terminal fragment of cystatin 3, or said N-terminal fragment of cystatin 3, is used in combination with one or more polypeptide(s) comprising N-terminal fragment of another cystatin, in particular of another type 2 cystatin, and/or with one or more N-terminal fragment(s) of another cystatin, in particular of another type 2 cystatin.

Similarly, the cystatin expression inducer may be administered in combination with at least one expression inducer of another cystatin for simultaneous, separate or sequential administration. In particular a cystatin 3 expression inducer may be administered in combination with at least one inducer of another cystatin, more preferably of another type 2 cystatin.

In some embodiments, the polypeptides and cystatin expression inducers according to the invention are provided in the form of pharmaceutical compositions further comprising a pharmaceutically acceptable vehicle, carrier, diluent, or excipient, or a mixture thereof. The pharmaceutical compositions according to the invention may be in particular in modified release dosage forms, and accordingly may further comprise one or more release controlling excipients as described herein. Suitable modified release dosage vehicles include, but are not limited to, hydrophilic or hydrophobic matrix devices, watersoluble separating layer coatings, osmotic devices, multiparticulate devices, and combinations thereof. The pharmaceutical compositions may also comprise non-release controlling excipients. The pharmaceutical compositions according to the invention may also be in a dosage form and have an instant releasing component and at least one delayed releasing component, and may be capable of giving a discontinuous release of the compound in the form of at least two consecutive pulses separated in time from 0.1 up to 24 hours.

The pharmaceutical compositions according to the invention may also be provided in unit-dosage forms or multiple-dosage forms. Unit-dosage forms, as used herein, refer to physically discrete units suitable for administration to subjects and packaged individually as is known in the art. Each unit-dose contains a predetermined quantity of the active ingredient(s) sufficient to produce the desired therapeutic effect, in association with the required pharmaceutical carriers or excipients. Examples of unit-dosage forms include ampoules, syringes, and individually packaged tablets, capsules and vaginal suppositories. Unit-dosage forms may be administered in fractions or multiples thereof. A multiple-dosage form is a plurality of identical unit-dosage forms packaged in a single container to be administered in segregated unit-dosage form. Examples of multiple-dosage forms include vials, bottles of tablets, capsules or vaginal suppositories, or bottles of pints or gallons.

The polypeptides and cystatin expression inducers described herein may be administered alone, or in combination with one or more other active ingredients. In particular, the polypeptides and cystatin expression inducers described herein may be administered in combination with one or more active ingredient used to treat sterility and/or infertility. Examples of active ingredients used to treat sterility and/or infertility are well-known from the skilled person and include for examples oestrogens; gonadotrophins; clomifen including clomid and pergotime; gonadotrophin releasing hormone analogues including gonadorelin, buserelin and triptorelin; luteinostimulin antagonists including cetrorelix and ganirelix.

The pharmaceutical compositions that comprise the polypeptides and/or cystatin expression inducers described herein may be formulated in various dosage forms, preferably for topical administration. The pharmaceutical compositions may also be formulated as modified release dosage forms, including delayed-, extended-, prolonged-, sustained-, pulsatile-, controlled-, accelerated-, fast-, targeted-, and programmed-release dosage forms. These dosage forms can be prepared according to conventional methods and techniques known to those skilled in the art ("Remington's Pharmaceutical Sciences", (l9th edition), ed. A. Gennaro, 1995, Mack Publishing Company, Easton, Pa.; Modified-Release Drug Deliver Technology, Rathbone et al., Eds., Drugs and the Pharmaceutical Science, Marcel Dekker, Inc.: New York, NY, 2002; Vol. 126).

The pharmaceutical compositions provided herein may be administered at once, or multiple times at intervals of time. It is understood that the precise dosage and duration of treatment may vary with the age, weight, and condition of the subject being treated, and may be determined empirically using known testing protocols or by extrapolation from *in vivo* or *in vitro* test or diagnostic data. It is further understood that for any particular subject, specific dosage regimens should be adjusted over time according to the subject need and the professional judgment of the person administering or supervising the administration of the formulations.

The polypeptides, cystatin expression inducers and pharmaceutical compositions according to the invention may be in particular administered orally, parenterally or topically (i.e. locally). Preferably, the polypeptides or cystatin expression inducers according to the invention are administered locally.

The polypeptides, cystatin expression inducers and pharmaceutical compositions according to the invention may be administered topically (or locally) to the orifices, or mucosa. The topical administration, as used herein, includes in particular mucosal, vaginal, urethral and uterine administration.

The pharmaceutical compositions provided herein may be formulated in any dosage forms that are suitable for topical administration for local effect, including emulsions, solutions, suspensions, creams, gels, hydrogels, ointments, dusting powders, dressings, elixirs, lotions, suspensions, tinctures, pastes, foams, films, aerosols, irrigations, sprays, suppositories, bandages, dermal patches. The topical formulation of the pharmaceutical compositions provided herein may also comprise liposomes, micelles, microspheres, nanosystems, and mixtures thereof. Pharmaceutically acceptable carriers and excipients suitable for use in the topical formulations provided herein include, but are not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, penetration enhancers, cryoprotectants, lyoprotectants, thickening agents, and inert gases.

The pharmaceutical compositions provided herein may be provided in the forms of ointments, creams, and gels, in particular in the forms of gynaecological ointments, creams and gels.

The pharmaceutical compositions provided herein may be administered mucosally, urethrally, vaginally, perivaginally or in the uterus in the forms of suppositories, pessaries, bougies, poultices or cataplasm, pastes, powders, dressings, creams, plasters, contraceptives, ointments, solutions, emulsions, suspensions, tampons, gels, foams, sprays, or enemas. These dosage forms can be manufactured using conventional processes as described in Remington: The Science and Practice of Pharmacy ("Remington's Pharmaceutical Sciences", (19th edition), ed. A. Gennaro, 1995, Mack Publishing Company, Easton, Pa.). Urethral and vaginal suppositories are solid bodies for insertion into body orifices, which are solid at ordinary temperatures but melt or soften at body temperature to release the active ingredient(s) inside the orifices. Pharmaceutically acceptable carriers utilized in vaginal suppositories include vehicles, such as stiffening agents, which produce a melting point in the proximity of body temperature, when formulated with the pharmaceutical compositions provided herein; and antioxidants as described herein, including bisulfite and sodium metabisulfite. Suitable vehicles include, but are not limited to, cocoa butter (theobroma oil), glycerin-gelatin, carbowax (polyoxyethylene glycol), spermaceti, paraffin, white and yellow wax, and appropriate mixtures of mono-, di- and triglycerides of fatty acids, hydrogels, such as polyvinyl alcohol, hydroxyethyl methacrylate, polyacrylic acid; glycerinated gelatin. Combinations of the various vehicles may be used. Vaginal suppositories may be prepared by the compressed method or molding. The typical weight of a vaginal suppository is about 2 to 3 g.

The pharmaceutical compositions provided herein for topical administration may be formulated to be immediate release or modified release, including delayed-, sustained-, pulsed-, controlled-, targeted-, and programmed release.

Preferably, the polypeptides and cystatin expression inducers according to the invention are administered in the form of a vaginal suppository.

The dosage of the polypeptides and cystatin expression inducers described herein may vary depending on the route of administration (oral, intravenous, vaginal or the like) and the form in which the composition or compound is administered (solution, controlled release or the like). Determination of appropriate dosages is within the ability of one of skill in the art.

As used herein, an "effective amount", a "therapeutically effective amount", or a "pharmacologically effective amount" of a medicament refers to an amount of a medicament present in such a concentration to result in a therapeutic level of drug delivered over the term that the drug is used. This may be dependent on mode of delivery, time period of the dosage, age, weight, general health, sex and diet of the subject receiving the medicament. Methods of determining effective amounts are known in the art.

In a preferred embodiment, the polypeptide comprising N-terminal fragment of a cystatin, preferably of a type 2 cystatin, more preferably of cystatin 3, and/or the cystatin expression inducer, preferably the type 2 cystatin expression inducer, more preferably the cystatin 3 expression inducer, according to the invention, is for use as a fertilization promoting or improving drug.

As used herein, the expression "fertilization promoting or improving drug" refers to a drug that induces or facilitates the meeting of a spermatozoon with an ovum, or induces or facilitates the entry of a spermatozoon into an ovum.

The present invention also relates to a method for promoting or improving fertilization in a subject in need thereof, comprising administering in said subject a therapeutically effective amount of (i) the polypeptide comprising N-terminal fragment of a cystatin, preferably of a type 2 cystatin, more preferably of cystatin 3, and/or of (ii) the cystatin expression inducer, preferably the type 2 cystatin expression inducer, more preferably the cystatin 3 expression inducer, according to the invention.

In another preferred embodiment, the polypeptide comprising N-terminal fragment of a cystatin, preferably of a type 2 cystatin, more preferably of cystatin 3, and/or the cystatin expression inducer, preferably the type 2 cystatin expression inducer, more preferably the cystatin 3 expression inducer, according to the invention, is for use for promoting or improving the fertilization or for promoting or improving the step of migration of a zygote from the fallopian tubes to the uterus in a female mammal.

As disclosed above, the polypeptide comprising N-terminal fragment of a cystatin may be administered in combination with at least one N-terminal fragment of another cystatin. Similarly, the cystatin expression inducer may be administered in combination with at least one expression inducer of another cystatin.

As used herein, the term "zygote" refers to the initial cell formed from the union of an ovum with a spermatozoon. After fertilization has taken place the zygote migrates down the fallopian tube to the uterus, while dividing to form more cells without the zygote actually increasing in size. Zygotes eventually develop into an embryo, and then a fetus.

The polypeptides and/or cystatin expression inducers according to the invention may be in particular useful for treating subjects with known problems of sterility and/or infertility, who need assisted reproductive technologies (ART) to reproduce.

Accordingly, in a particular embodiment, the polypeptide comprising N-terminal fragment of a cystatin, preferably of a type 2 cystatin, more preferably of cystatin 3, and/or the cystatin expression inducer, preferably the type 2 cystatin expression inducer, more preferably the cystatin 3 expression inducer, according to the invention, is for use for promoting or improving the fertilization or for promoting or improving the step of migration of a zygote from the fallopian tubes to the uterus in a female mammal, wherein said female mammal is a mammal turning to assisted reproductive technologies (ART).

As used herein, the expression "assisted reproductive technologies" or ART refers to methods used to achieve pregnancy by artificial or partially artificial means.

Assisted reproductive technologies include in particular *in vitro* fertilization and extensions to it, but not necessary. As used herein, assisted reproductive technologies encompass *in vitro* fertilization, autologous endometrial coculture, zygote intrafallopian transfer (ZIFT), gamete intrafallopian transfer (GIFT), artificial insemination in particular therapeutic donor insemination (TDI).

As used herein, the expression *"in vitro* fertilization" or "IVF" refers to the technique of letting fertilization of the male and female gametes occur outside the female body.

As used herein, the term "autologous endometrial coculture" refers to a treatment wherein the patient's fertilized eggs are placed on top of a layer of cells from the patient's own uterine lining, creating a more natural environment for embryo development.

As used herein, the term "zygote intrafallopian transfer" or "ZIFT" refers to the technology wherein ova are removed from the female's ovaries and fertilized *in vitro;* the resulting zygote being then placed into the fallopian tube.

As used herein, the term "gamete intrafallopian transfer" or "GIFT" refers to the technology wherein a mixture of sperm and ova is placed directly into a female's fallopian tube using laparoscopy following a transvaginal ovum retrieval.

As used herein, the term "artificial insemination" refers to the technology wherein sperm is placed into a female's uterus (intrauterine) or cervix (intracervical) using artificial means rather than by natural copulation. "Therapeutic donor insemination" or "TDI" refers more particularly to the case wherein the female does not have a partner with functional sperm. Instead, a sperm donor supplies the sperm.

Preferably, the polypeptide comprising N-terminal fragment of a cystatin, preferably of a type 2 cystatin, more preferably of cystatin 3, and/or the cystatin expression inducer, preferably the type 2 cystatin expression inducer, more preferably the cystatin 3 expression inducer, according to the invention, is for use for promoting or improving the fertilization or for promoting or improving the step of migration of a zygote from the fallopian tubes to the uterus in a female mammal, wherein said female mammal is a mammal undergoing gamete intrafallopian transfer procedure (GIFT) or therapeutic donor insemination (TDI), or is a mammal turning to *in vitro* fertilization (IVF), during assisted reproductive technologies (ART).

### Method of in vitro fertilization and/or artificial insemination

The present invention also relates to a method of *in vitro* fertilization and/or artificial insemination comprising the steps of contacting an ovum obtained from a female mammal and a spermatozoon obtained from a male mammal in a fluid medium comprising a polypeptide comprising N-terminal fragment of a cystatin, preferably of a type 2 cystatin, more preferably of cystatin 3, as defined above.

In a particular embodiment, said fluid medium comprises several polypeptides, each comprising N-terminal fragment of a different cystatin.

As used herein, the term "ovum" or "ova" refers to a haploid female reproductive cell or gamete. In assisted reproductive technologies, ova may be recovered from the female, for example by transvaginal ovum retrieval (OCR).

As used herein, the term "sperm" or "spermatozoon" or "spermatozoa" refers to the male reproductive cells.

As used herein, the term "fluid medium" refers to a biological medium which is in a liquid or semi-liquid form. Preferably, the fluid medium according to the invention refers to a medium wherein ova and sperm are able to survive, at least for the same period as in normal conditions inside the individual's body. The fluid medium according to the invention may be an artificial fluid medium or a natural fluid medium. When the fluid medium is a natural fluid medium, it may be in particular the fluid medium present in the fallopian tubes or in the uterus. When the fluid medium is an artificial fluid, it may be in particular an *in vitro* fertilization medium. I*n vitro* fertilization media are well-known from the skilled person and include for example Universal IVF medium^{®} (commercialized by Origio), global^{®} for fertilization (commercialized by LifeGlobal) and Sydney IVF media (commercialized by Sydney IVF).

The fluid medium according to the invention comprises a polypeptide comprising N-terminal fragment of a cystatin, preferably of a type 2 cystatin, more preferably of cystatin 3, as defined above. Preferably, said polypeptide comprising N-terminal fragment of a cystatin is a cystatin protein, preferably a type 2 cystatin protein, more preferably the cystatin 3 protein. In a particular embodiment, the fluid medium may further comprise at least one N-terminal fragment of another cystatin. In particular, the fluid medium may comprise a polypeptide comprising N-terminal fragment of cystatin 3 or the N-terminal fragment of cystatin 3 and further comprise one or more polypeptide(s) comprising N-terminal fragment of another cystatin, in particular of another type 2 cystatin, and/or one or more N-terminal fragments of another cystatin, in particular of another type 2 cystatin.

In a preferred embodiment, said polypeptide comprising N-terminal fragment of a cystatin is present in the fluid medium according to the invention at an adequate concentration around the physiological one.

In a particular embodiment, the fluid medium according to the invention further comprises compounds that are useful for treating sterility and/or infertility, as defined above.

As used herein, the term "contacting" means incubating ova with spermatozoa such that spermatozoa have the possibility to fertilize the ova. Typically, the ova and the spermatozoa are contacted at an adequate temperature preferably during an adequate period of time, according to the skill of the art.

### Method of diagnosing and/or predicting sterility and/or infertility

The present invention also relates to a method, preferably an *in vitro* method, of diagnosing and/or predicting sterility and/or infertility of a subject and/or of a couple of subjects comprising the steps of:
(a1) measuring the level of at least one cystatin in a biological sample of a male subject, wherein said sample is selected from the group consisting of blood, plasma, serum, semen, sperm and seminal plasma, and/or
(a2) measuring the level of at least one cystatin in a biological sample of a female subject, wherein said sample is selected from the group consisting of blood, plasma, serum, cervical mucus and vaginal mucus and
(b) comparing the level of the at least one cystatin measured in step (a1) and/or (a2) with a male control level of said at least one cystatin or female control level of said at least one cystatin, as appropriate;
wherein a decreased level of the at least one cystatin measured in step (a1) and/or (a2) compared to said male control level of said at least one cystatin or female control level of said at least one cystatin is indicative of sterility and/or infertility of said subject and/or couple of subjects, or is indicative of a higher risk of sterility and/or infertility of said subject and/or couple of subjects.

In a preferred embodiment, the step (a1) is a step of measuring the level of at least one type 2 cystatin in a biological sample of a male subject as defined above.

In another preferred embodiment, the step (a2) is a step of measuring the level of at least one type 2 cystatin in a biological sample of a female subject as defined above.

In a more preferred embodiment, the step (a1) is a step of measuring the level of at least cystatin 3 in a biological sample of a male subject as defined above.

In another more preferred embodiment, the step (a2) is a step of measuring the level of at least cystatin 3 in a biological sample a female subject as defined above.

In a particular embodiment, the step (a1) is a step of measuring the level of several different cystatins, more particularly of cystatin 3 and at least another cystatin, in a biological sample of a male subject as defined above.

In another particular embodiment, the step (a2) is a step of measuring the level of several different cystatins, more particularly of cystatin 3 and at least another cystatin, in a biological sample of a female subject as defined above.

As intended herein, the term "diagnosis" or "diagnosing" relates to a method for determining the pathology afflicting a patient.

As intended herein, the term "prediction" or "predicting" relates to a method for predicting the appearance of a pathology or for determining the risk or likeliness that a pathology will occur in an individual.

As used herein, the "level" of a cystatin corresponds to the concentration, the amount or the activity of a cystatin. Preferably, the level of a cystatin refers to the amount or concentration of a cystatin, more particularly to the molar or the mass amount or to the molar or the mass concentration of a cystatin.

Techniques to measure the level of a cystatin in a biological sample are well-known from one skilled in the art. Examples of suitable techniques include mass spectrometry, but also ligand-based methods such as immunological assays. In particular the level of a cystatin is measured using ligands specific for said cystatin.

A "ligand" relates to a molecule which has binding affinity towards a compound. In particular it is preferred that the ligand binds to a cystatin in a specific manner, that is the ligand preferentially binds to the compound it is specific for as compared to other components of the biological sample. The specific ligands are in particular selected from the group consisting of polyclonal, monoclonal and recombinant antibodies, or fragments thereof, such as Fab fragments, F(ab')₂ fragments and scFv fragments; phage antibodies (PhAbs); Ilama, camel and shark antibodies; and aptamers.

The expression "biological sample" encompasses all samples which can be taken from a subject, such as tissue biopsies or samples of body fluids. In particular, the biological sample may be a liquid biological sample. The biological sample may thus be selected from the group consisting of blood sample, serum and plasma. The biological sample may also be a sample from a fluid of the reproductive tract. More particularly, the biological sample of a male subject may be selected from blood, plasma and serum, but also semen, sperm and seminal plasma. Similarly, a biological sample of a female subject may be selected from blood, plasma and serum, but also cervical mucus and vaginal mucus.

As used herein, the term "semen" refers to an organic fluid, also known as seminal fluid, which may contain spermatozoa. The semen is typically constituted of three fractions, namely non-germinal mononucleated cells (including leucocytes, desquamated cells from the walls of the genital tract and immature germinal cells), spermatozoa (or sperm) and seminal plasma.

As used herein, the term "seminal plasma" refers to the liquid secreted by seminal vesicles that is included in semen and that comprises ascorbic acid, fructose, prostaglandine, phosphorylcholine and vesiculin.

As used herein, the term "cervical mucus" refers to the cervical glar produced by the uterus cervix.

As used herein, the term "vaginal mucus" refers to the glar produced by the vagina.

As intended herein a "cystatin control level" refers to a level of a cystatin which is within the norm cut-off values for said cystatin. Said cystatin control level is dependent on the biological sample type and on the method used for measuring the level of said cystatin in the biological sample. Preferably, the cystatin control level is the mean level of expression of said cystatin in a healthy population. More particularly, the "male cystatin control level" according to the invention is the mean level of expression of said cystatin in a healthy male population. Similarly, the "female cystatin control level" according to the invention is the mean level of expression of said cystatin in a healthy female population.

Typically, when the level of cystatin 3 is measured in a male biological sample which is a sample of seminal plasma, using antibodies specific for cystatin 3, in particular those of Dade Behring, Dako or Diagam, the CST3 control level is about 30 ng/ml.

As used herein, a "healthy population" means a population constituted of subjects who do not present problems of sterility and/or infertility.

Preferably, in the methods of diagnosis and/or prediction of the invention, a decreased level of a cystatin compared to the control level of said cystatin means that said level of cystatin is significantly lower than the control level of said cystatin. Still preferably, a decreased level of a cystatin compared to the control level of said cystatin means that said level of cystatin is 75%, 50% the control level of said cystatin.

The present inventors have more particularly demonstrated that a decrease of cystatin 3 concentration either in male or female could lead to infertility. More specifically, they showed that a too low combined concentration of cystatin 3 in the male and the female may be responsible for infertility.

Accordingly, in a preferred embodiment, the method, preferably the *in vitro* method, of diagnosis and/or prediction according to the invention is preferably a method of diagnosing and/or predicting sterility and/or infertility of a couple of subjects and comprises the steps of:
(a1) measuring the level of at least one cystatin in the biological sample of the male subject as defined above,
(a2) measuring the level of at least one cystatin in the biological sample of the female subject as defined above;
(a3) adding the level of the at least one cystatin measured in step (a1) and the level of the at least one cystatin measured in step (a2); and
(b) comparing the level of the at least one cystatin obtained in step (a3) with a control level of said at least one cystatin;
wherein a decreased level of the at least one cystatin obtained in step (a3) compared to the control level of said at least one cystatin is indicative of sterility and/or infertility of said couple of subjects, or is indicative of a higher risk of sterility and/or infertility of said couple of subjects.

In a preferred embodiment, the step (a1) is a step of measuring the level of at least one type 2 cystatin in a biological sample of a male subject as defined above.

In another preferred embodiment, the step (a2) is a step of measuring the level of at least one type 2 cystatin in a biological sample of a female subject as defined above.

In a more preferred embodiment, the step (a1) is a step of measuring the level of at least cystatin 3 in a biological sample of a male subject as defined above.

In another more preferred embodiment, the step (a2) is a step of measuring the level of at least cystatin 3 in a biological sample a female subject as defined above.

In a particular embodiment, the step (a1) is a step of measuring the level of several different cystatins, more particularly of cystatin 3 and at least another cystatin, in a biological sample of a male subject as defined above.

In another particular embodiment, the step (a2) is a step of measuring the level of several different cystatins, more particularly of cystatin 3 and at least another cystatin, in a biological sample of a female subject as defined above.

Preferably, the control level of a cystatin is the mean level of expression of said cystatin in a healthy population of couples.

As used herein, a "healthy population of couples" means a population constituted of couples having intercourses who do not present problems of sterility and/or infertility.

### Kit

The present invention also relates to a kit intended for diagnosing and/or predicting sterility and/or infertility of a subject and/or of a couple of subjects comprising:
(i) means for detecting at least one cystatin, in particular at least one type 2 cystatin, more particularly at least cystatin 3 in a biological sample of a male subject, wherein said sample is selected from the group consisting of blood, plasma, serum, semen, sperm and seminal plasma and/or
(ii) means for detecting at least one cystatin, in particular at least one type 2 cystatin, more particularly at least cystatin 3 in a female biological sample of a female subject, wherein said sample is selected from the group consisting of blood, plasma, serum, cervical mucus and vaginal mucus.

In the context of the invention, the expression "means for detecting a cystatin" refers in particular to ligands specific of a cystatin as defined above.

Preferably, the kit of the invention further comprises instructions for using the means for detecting at least one cystatin included in the kit as defined above to carry out the diagnosis and/or prediction of sterility and/or infertility.

"Instructions for use" typically include a tangible expression describing the technique to be employed in using the components of the kit and explaining the conditions to be used to carry out the diagnosis and/or prediction of sterility and/or infertility.

### Likelihood of benefit from a treatment

The present invention also relates to a method of determining whether a subject and/or a couple of subjects suffering from sterility and/or infertility is likely to benefit from a treatment regimen that includes treatment with (i) a polypeptide comprising N-terminal fragment of a cystatin, preferably of a type 2 cystatin, more preferably of cystatin 3 and/or with (ii) a cystatin expression inducer, preferably a type 2 cystatin expression inducer, more preferably a cystatin 3 expression inducer, as defined above comprising:
(a1) measuring the level of a cystatin, preferably of a type 2 cystatin, more preferably of cystatin 3 in a biological sample of a male subject, wherein said sample is selected from the group consisting of blood, plasma, serum, semen,
sperm and seminal plasma as defined above in the section *"Method of diagnosing and*/*or predicting sterility andlor infertility"* and/or
(a2) measuring the level of a cystatin, preferably of a type 2 cystatin, more preferably of cystatin 3 in a biological sample of a female subject, wherein said sample is selected from the group consisting of blood, plasma, serum, cervical mucus and vaginal mucus as defined above in the section *"Method of diagnosing and*/*or predicting sterility and*/*or infertility".*

As used herein, the term "likely to benefit" means that the subjects have an increased probability of presenting a higher fertility as compared to subjects suffering from sterility and/or infertility who do not receive a treatment with a polypeptide comprising N-terminal fragment of a cystatin and/or with a cystatin expression inducer.

As used herein, the term "treatment regimen" refers to any systematic plan or course for treating a disease in a subject.

The present invention also relates to a method, in particular an *in vitro* method, of determining whether a subject and/or a couple of subjects suffering from sterility and/or infertility is likely to benefit from a treatment regimen that includes treatment with (i) a polypeptide comprising N-terminal fragment of a cystatin, preferably of a type 2 cystatin, more preferably of cystatin 3 and/or with (ii) a cystatin expression inducer, preferably a type 2 cystatin expression inducer, more preferably a cystatin 3 expression inducer, as defined above comprising:
(a1) measuring the level of a cystatin, preferably of a type 2 cystatin, more preferably of cystatin 3 in a biological sample of a male subject, wherein said sample is selected from the group consisting of blood, plasma, serum, semen, sperm and seminal plasma as defined above in the section *"Method of diagnosing andlor predicting sterility and*/*or infertility"* and/or
(a2) measuring the level of a cystatin, preferably of a type 2 cystatin, more preferably of cystatin 3 in a biological sample of a female subject, wherein said sample is selected from the group consisting of blood, plasma, serum, cervical mucus and vaginal mucus as defined above in the section *"Method of diagnosing andlor predicting sterility and*/*or infertility";*
(b) comparing the level of said cystatin measured in step (a1) and/or (a2) with a male control level of said cystatin or female control level of said cystatin as defined above in the section *"Method of diagnosing andlor predicting sterility andlor infertility";* as appropriate;
wherein a decreased level of the cystatin measured in step (a1) and/or (a2) compared to said male control level of said cystatin or female control level of said cystatin indicates that the subject and/or the couple of subjects is likely to benefit from the treatment regimen that includes treatment with a polypeptide comprising N-terminal fragment of a cystatin and/or with a cystatin expression inducer as defined above.

The present inventors have more particularly demonstrated that a decrease of cystatin 3 concentration either in male or female could lead to infertility. More specifically, they showed that a too low combined concentration of cystatin 3 in the male and the female may be responsible for infertility.

Accordingly, in a preferred embodiment, the method defined above is for determining whether a couple of subjects suffering from sterility and/or infertility is likely to benefit from a treatment regimen that includes treatment with (i) a polypeptide comprising N-terminal fragment of a cystatin, preferably of a type 2 cystatin, more preferably of cystatin 3, and/or with (ii) a cystatin expression inducer, preferably a type 2 cystatin expression inducer, more preferably a cystatin 3 expression inducer, as defined above and comprises:
(a1) measuring the level of a cystatin, preferably of a type 2 cystatin, more preferably of cystatin 3, in a biological sample of a male subject as defined above;
(a2) measuring the level of a cystatin, preferably of a type 2 cystatin, more preferably of cystatin 3, in a biological sample of a female subject as defined above;
(a3) adding the level of the cystatin measured in step (a1) and the level of the cystatin measured in step (a2); and
(b) comparing the level of said cystatin obtained in step (a3) with a control level of said cystatin as defined above in the section *"Method of diagnosing and*/*or predicting sterility and*/*or infertility";*
wherein a decreased level of the cystatin obtained in step (a3) compared to said the control level of said cystatin indicates that the couple of subjects is likely to benefit from the treatment regimen that includes treatment with a polypeptide comprising N-terminal fragment of a cystatin and/or with a cystatin expression inducer as defined above.

The invention will be further illustrated by the following figure and example.

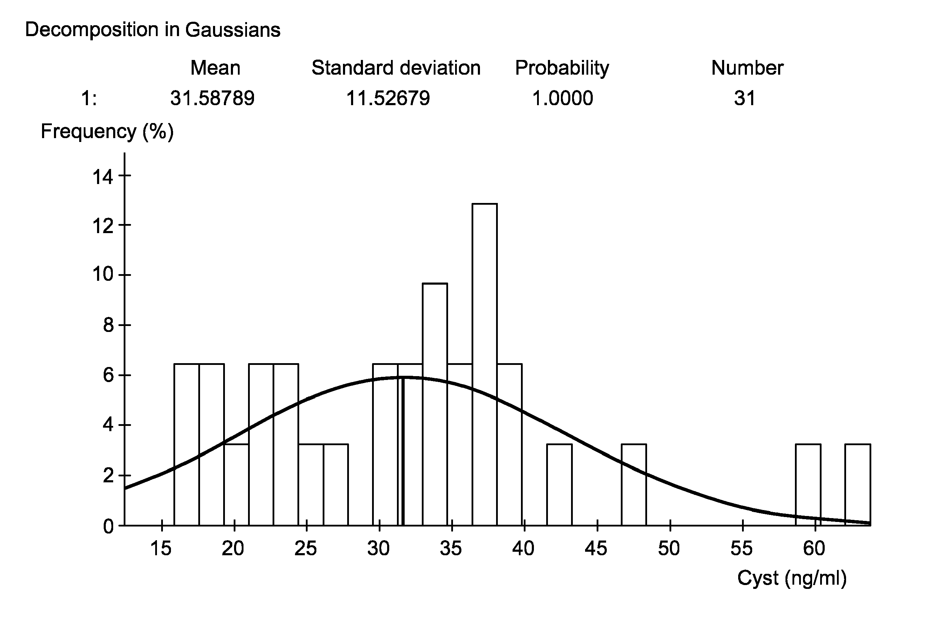

### Description of the figure

The **Figure** displays the distribution of the cystatin C concentration in the seminal fluid of 31 sterile men, as described in the Example.

### Example

The following example demonstrates that sterile men present a decreased level of cystatin 3 in their seminal fluid.

### Materials and methods

### Cystatin C assays were performed by immunoassay using the BNII immunonephelemeter (Dade Behring)

### BNII immunonephelometer

The immunonephelemeter is an automaton driven by a computer developed by Dade Behring, enabling measuring the intensity of the light spread by the antigen-antibody complexes by the following way:

A light beam with a wave length of 840 nm is generated by a laser diode and sent to the chamber where the antigen-antibody reaction occurs. This beam crosses this chamber, wherein it is scattered. The scattered and filtrated light at the end of the chamber is focalized with a complex of lenses on the photodetector, which transforms it into an electric signal.

The measure of the scattered light is done according to the principle of kinetics in fixed time: a first measurement is performed 10 seconds after initialization, and a second measurement is performed after 6 minutes of incubation.

The light scattered by the antigen-antibody complexes may be measured:
- in a final point at the end of the reaction;
- in a kinetic measure, continually during the reaction, but the calculation will be done by the difference between the signals measured at two time points fixed in the time, *i.e.* a measure in fixed time. This type of measure is used here. The blank value corresponds to the first value measured after 7.5 seconds. The second value is measured after a 6.12 to 24 minutes incubation.

For performing such an assay, a calibration curve is necessary. It is obtained with standards specific of the test, but since there is no linear relation between the measured signal and concentration of the protein to be tested, different measurement points have to be taken to obtain a curve.

A Heidelberger-Kendall curve is also used as a reference curve to show the relation between the amount of antigen and the measured signal for a constant concentration of antibodies.

### Reagents

- cystatin C reagent: lyophilizate of polystyrene particles, coated with rabbit anti-cystatin C antibodies.
- cystatin C supplementary A reagent: contains rabbit immunoglobulins in a buffer. It enables reducing interferences due to rheumatoid factors.
- cystatin C supplementary B reagent: contains a detergent.
- cystatin C control: lyophilizate obtained from polygelins, to which human urinary proteins were added.
- UY protein standard
- diluent
- Dade Behring buffer.

### Preparation of the reagents

- cystatin C reagent: the lyophilized content of the vial was reconstituted with the distilled water volume of the kit. After stirring, it was left 30 min before use.
- cystatin C supplementary reagent: a volume of 0.5 ml of cystatin C supplementary B reagent was added to the vial of cystatin C supplementary A reagent. The solution was mixed before use.
- cystatin C control: the lyophilized content of the vial was prepared 30 min before use by being reconstituted in 1 ml of distilled water and homogenised.

### Samples preparation

The semen samples were diluted 1/40 in Dade buffer. They were then automatically diluted in the immunonephelemeter with a dilution of 1/20.

### Measurement of the cystatin C amount in the seminal fluid

The results obtained with the immunonephelemeter, expressed in mg/l, were multiplied by 40, then by the volume of sperm taken from the patient. The total amount of cystatin C in the semen was thus obtained.

### Statistical analysis

The results obtained were analyzed with the statistics software STATFRA 2005 (Alcatel TITN Answare).

Correlations were examined using the Spearman correlation test.

### Results

### Cystatin C concentrations values

Cystatin C assays were performed on 31 male sterile patients, among whom 18 had a seminal biochemical analysis.

**Table 1** displays the cystatin C concentrations in the seminal fluid of the 31 sterile patients. **Table 2** displays the cystatin C concentrations in the seminal fluid of the 18 sterile patients who underwent a seminal biochemical analysis.

**Table 1: Mean values of cystatin C concentrations and total amounts in the seminal fluid obtained from the semen of sterile men**

| **N = 31 patients** | **Cystatin C concentration (ng/ml)** | **Total amount of cystatin C (ng) *** |
|---|---|---|
| Mean ± Standard deviation | 31.58 ± 11.72 | 143.18 ± 70.10 |

| | | |
|---|---|---|
| *: the total amount of cystatin C (ng) was obtained by multiplying its concentration (ng/ml) by the ejaculate volume of the patient. | | |

**Table 2: Mean values of cystatin C concentrations and total amounts in the seminal fluid obtained from the semen of sterile men who underwent a seminal biochemical analysis.**

| **N = 18 patients** | **Mean** | **Standard deviation** |
|---|---|---|
| Cystatin C concentration | 31.13 | 9.36 |
| (ng/ml) | | |
| Total amount of cystatin C | 129.17 | 56.06 |
| (ng) | | |
| Total amount of fructose | 73.72 | 38.03 |
| (µmoles) | | |
| Total amount of acid phosphatase (U.10³) | 108.44 | 61.92 |
| Total amount of citrate | 100.11 | 46.55 |
| (µmoles) | | |
| Total amount of zinc | 19.26 | 13.44 |
| (µmoles) | | |
| Total amount of carnitine | 896.22 | 584.84 |
| (nmoles) | | |

**Table 3** displays the cystatin C concentration in a fractionated ejaculate of semen of a fertile man.

**Table 3: Concentrations of cystatin C, sperm and zinc in a fractionated ejaculate of a normal man**

| **Fraction number** | **Cystatin C concentration (ng/ml)** | **Sperm concentration (10⁸ spermatozoa/ml)** | **Zinc concentration (nmol/ml)** |
|---|---|---|---|
| 1 | 31.2 | 3.10 | 5.09 |
| 2+3 (pool) | 33.6 | 1.68 | 3.154 |
| 4 | 30.4 | 0.63 | 2.37 |
| 5 | 34 | 0.39 | 2.36 |
| 6 | 33.6 | 0.34 | 1.43 |

The distribution of cystatin C on the population of 31 sterile patients shows a repartition into 3 groups (**Figure**).

A majority of the patients have a cystatin C concentration of between 30 and 40 ng/ml. The two other groups of patients display a cystatin C concentration of between 15 and 30 ng/ml or a cystatin C concentration superior to 60 ng/ml.

## Claims

1. A polypeptide comprising N-terminal fragment of a cystatin, and/or a cystatin expression inducer for use for the treatment of sterility and/or infertility.

2. The polypeptide comprising N-terminal fragment of a cystatin and/or cystatin expression inducer for its use according to claim 1, wherein said cystatin is cystatin 3.

3. The polypeptide comprising N-terminal fragment of a cystatin and/or cystatin expression inducer for its use according to claim 1 or 2, wherein the polypeptide comprising N-terminal fragment of a cystatin is administered in combination with at least one N-terminal fragment of another cystatin.

4. The polypeptide comprising N-terminal fragment of a cystatin and/or cystatin expression inducer for its use according to any one of claims 1 to 3, for treating male and/or female sterility and/or infertility.

5. The polypeptide comprising N-terminal fragment of a cystatin and/or cystatin expression inducer for its use according to any one of claims 1 to 4, for treating sterility and/or infertility of a couple of subjects.

6. The polypeptide comprising N-terminal fragment of a cystatin and/or cystatin expression inducer for its use according to any one of claims 1 to 5, wherein said polypeptide or cystatin expression inducer is administered locally.

7. A polypeptide comprising N-terminal fragment of a cystatin, and/or cystatin expression inducer, for use as a fertilization promoting or improving drug.

8. A polypeptide comprising N-terminal fragment of a cystatin and/or cystatin expression inducer for use for promoting or improving the step of migration of a zygote from the fallopian tubes to the uterus in a female mammal.

9. The polypeptide comprising N-terminal fragment of a cystatin and/or cystatin expression inducer for its use according to claim 8, wherein said female mammal is a mammal undergoing gamete intrafallopian transfer procedure (GIFT) or therapeutic donor insemination (TDI), or is a mammal turning to *in vitro* fertilization (IVF), during assisted reproductive technologies (ART).

10. A method of *in vitro* fertilization and/or artificial insemination comprising the steps of contacting an ovum obtained from a female mammal and a spermatozoon obtained from a male mammal in a fluid medium comprising a polypeptide comprising N-terminal fragment of a cystatin.

11. The method according to claim 10, wherein said cystatin is cystatin 3.

12. An *in vitro* method of diagnosing and/or predicting sterility and/or infertility of a subject and/or of a couple of subjects comprising the steps of:
(a1) measuring the level of at least one cystatin in a biological sample of a male subject, wherein said sample is selected from the group consisting of blood, plasma, serum, semen, sperm and seminal plasma and/or
(a2) measuring the level of at least one cystatin in a biological sample of a female subject, wherein said sample is selected from the group consisting of blood, plasma, serum, cervical mucus and vaginal mucus; and
(b) comparing the level of the at least one cystatin measured in step (a1) and/or (a2) with a male control level of said at least one cystatin or female control level of said at least one cystatin, as appropriate;
wherein a decreased level of the at least one cystatin measured in step (a1) and/or (a2) compared to said male control level of said at least one cystatin or female control level of said at least one cystatin is indicative of sterility and/or infertility of said subject and/or couple of subjects, or is indicative of a higher risk of sterility and/or infertility of said subject and/or couple of subjects.

13. The method according to claim 12, comprising the steps of:
(a1) measuring the level of at least one cystatin in the biological sample of the male subject as defined in claim 12,
(a2) measuring the level of at least one cystatin in the biological sample of the female subject as defined in claim 12;
(a3) adding the level of said at least one cystatin measured in step (a1) and the level of said at least one cystatin measured in step (a2); and
(b) comparing the level of said at least one cystatin obtained in step (a3) with a control level of said at least one cystatin;
wherein a decreased level of said at least one cystatin obtained in step (a3) compared to said control level of said at least one cystatin is indicative of sterility and/or infertility of said couple of subjects, or is indicative of a higher risk of sterility and/or infertility of said couple of subjects.

14. A kit intended for diagnosing and/or predicting sterility and/or infertility of a subject and/or of a couple of subjects comprising:
(i) means for detecting at least one cystatin in a biological sample of a male subject, wherein said sample is selected from the group consisting of blood, plasma, serum, semen, sperm and seminal plasma and/or
(ii) means for detecting at least one cystatin in a biological sample of a female subject, wherein said sample is selected from the group consisting of blood, plasma, serum, cervical mucus and vaginal mucus.

15. A method of determining whether a subject and/or a couple of subjects suffering from sterility and/or infertility is likely to benefit from a treatment regimen that includes treatment with a ipolypeptide comprising N-terminal fragment of a cystatin and/or with a cystatin expression inducer comprising:
(a1) measuring the level of a cystatin in a biological sample of a male subject, wherein said sample is selected from the group consisting of blood, plasma, serum, semen, sperm and seminal plasma and/or
(a2) measuring the level of a cystatin in a biological sample of a female subject, wherein said sample is selected from the group consisting of blood, plasma, serum, cervical mucus and vaginal mucus.
